(19) Europäisches Patentamt
European Patent Office
Office européen des brevets

(11) Numéro de publication : **0 060 954**
**B1**

# (12) FASCICULE DE BREVET EUROPÉEN

(45) Date de publication du fascicule du brevet : **15.08.84**

(51) Int. Cl.[3] : **C 07 C 91/16**, C 07 C 97/10, A 61 K 31/12, A 61 K 31/135

(21) Numéro de dépôt : **81400451.1**

(22) Date de dépôt : **23.03.81**

(54) **Dérivés de fluorophénacyl-amine, et leur application en thérapeutique.**

(43) Date de publication de la demande :
**29.09.82 Bulletin 82/39**

(45) Mention de la délivrance du brevet :
**15.08.84 Bulletin 84/33**

(84) Etats contractants désignés :
**AT BE CH DE GB IT LI LU NL SE**

(56) Documents cités :
**FR-A- 2 081 326**
**CHEMICAL ABSTRACTS, vol. 55, no. 23, 13 novembre 1961, réf. no. 23808 et réf. no. 23809a, COLUMBUS OHIO (US)**
**CHEMICAL ABSTRACTS, vol. 51-55, 1957-1961, Sixth Collective Index, Formules A-C12, COLUMBUS OHIO (US), page 998**
**IL FARMACO, Ed. Scientifica, vol. 24, no. 3, mars 1969 L. VILLA et al.: "Sintesi e porprieta farmacologiche di N-isopropilfeniletanolamine orto, meta o Para-sostituite", pages 329-340**
**CHEMICAL ABSTRACTS, vol. 47, no. 4, 25 février 1953, réf. no. 1839b COLUMBUS OHIO (US)**

(73) Titulaire : **LABORATOIRE L. LAFON Société anonyme dite:**
**1 rue Georges Médéric**
**F-94701 Maisons-Alfort (FR)**

(72) Inventeur : **Lafon, Louis**
**5 rue de l'Alboni**
**F-75016 Paris (FR)**

(74) Mandataire : ***Combe, André et al***
**CABINET BEAU DE LOMENIE 55 rue d'Amsterdam**
**F-75008 Paris (FR)**

**EP 0 060 954 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a trait, en tant que produits industriels nouveaux, à des dérivés de fluorophénacyl-amine. Elle concerne également leur application en thérapeutique.

Dans ce qui suit, par dérivés de fluorophénacylamine, on entend non seulement des composés présentant un groupe fluorophénacyle de formule $F—C_6H_4—CO—CH_2—$, mais encore un groupe β-hydroxy-fluorophénéthyle de formule $F—C_6H_4—CHOC—CH_2—$ qui dérive du précédent par réduction de la fonction carbonyle en fonction alcool.

Des composés du type 2-amino-1-(halogénophényl)-1-éthanol sont inclus dans la formule du brevet français n° 1 503 517 et présentés comme agents antidiurétiques. Or, il convient de remarquer que ce brevet français ne décrit aucun dérivé 1-(fluorophényl), 1-(chlorophényl), 1-(bromophényl) et 1-(iodophényl), ni ne suggère leurs actions potentielles sur le SNC.

On sait que l'on a déjà décrit des dérivés de fluorophénacyl-amine appartenant à la famille des 2-amino-1-(fluorophényl)-1-éthanols. On connaît en particulier de l'article de A.M. LANDS, J. Pharmacol. Exptl. Therap. 106, 440-443 (1952), le 1-(3-fluorophényl)-2-isopropylamino-1-éthanol et le 1-(3-fluorophényl)-2-tertiobutylamino-1-éthanol, et de l'article de L. VILLA et al., Il Farmaco Ed. Scientifica, 24 (n° 3), 329-340 (1969), le 1-(4-fluorophényl)-2-isopropylamino-1-éthanol et le 1-(2-fluorophényl)-2-isopropylamino-1-éthanol. Ces produits fluorés connus agissent sur le SNC mais présentant, en particulier, l'inconvénient d'être soit dénués d'effet antiagressif, soit faiblement antiagressifs.

On vient de trouver de façon surprenante que de nouveaux dérivés de fluorophénacyl-amine, qui agissent sur le SNC, possèdent des propriétés antiagressives particulièrement intéressantes sur le plan thérapeutique.

Selon l'invention, on préconise, en tant que produit industriel nouveau, utile notamment en thérapeutique, un composé appartenant à l'ensemble des dérivés de fluorophénacyl-amine de formule :

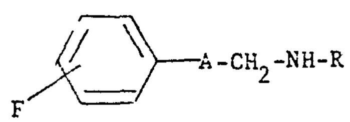

(I)

où A représente CO ou CHOH, et R est $CH(CH_3)_2$ ou $C(CH_3)_3$, ledit composé étant caractérisé en ce qu'il est choisi parmi l'ensemble constitué par les N-(4-fluorophénacyl)-isopropylamine, N-(2-fluorophénacyl)-tertiobutylamine, 1-(2-fluorophényl)-2-tertiobutylamino-1-éthanol, 1-(4-fluorophényl)-2-tertiobutylamino-1-éthanol, et leurs sels d'addition.

Parmi ces produits, les composés préférés sur le plan thérapeutique sont la N-(4-fluorophénacyl)-isopropylamine et ses sels, notamment le chlorhydrate.

Par sels d'addition, on entend ici les sels d'addition d'acides obtenus par réaction d'une base libre de formule I avec un acide minéral ou organique, et les sels d'ammonium. Parmi les acides utilisables pour salifier les bases de formule I, on peut notamment citer les acides chlorhydrique, bromhydrique, nitrique, sulfurique, acétique, propionique, oxalique, formarique, maléique, succinique, benzoïque, cinnamique, mandélique, citrique, malique, tartrique, p-toluènesulfonique et méthanesulfonique. Parmi les composés permettant d'obtenir des sels d'ammonium, on peut citer notamment $ICH_3$ et $ClCH_3$. Les sels d'addition d'acides sont les sels préférés, et parmi ces derniers, les plus intéressants sont les chlorhydrates.

Les dérivés de fluorophénacyl-amine de formule I peuvent être préparés selon une méthode connue en soi, par application de mécanismes réactionnels classiques. Le procédé de préparation que l'on préconise consiste :

1. à obtenir un composé « carbonyle » (A = CO) par réaction d'un halogénure de fluorophénacyle de formule :

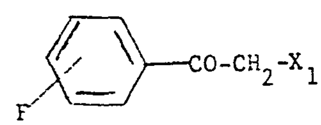

(II)

(où $X_1$ représente Cl ou Br)
avec une amine de formule :

$$H_2NR \qquad (III)$$

(où R est défini comme ci-dessus), au reflux pendant au moins 1 h, dans un alcool, le méthanol de préférence, puis

2. le cas échéant, à obtenir un composé « alcool » (A = CHOH) en réduisant le dérivé carbonyle correspondant, notamment au moyen de $NaBH_4$.

Les composés selon l'invention sont tous actifs sur le SNC et présentent en outre des effets

cardiovasculaires intéressants. Ils agissent en particulier sur le SNC en tant qu'agents sédatifs, antidépresseurs et antiagressifs, et sont indiqués dans le traitement des dépressions.

Selon l'invention, on préconise une composition thérapeutique, utile notamment dans le traitement des dépressions, caractérisée en ce qu'elle renferme, en association avec un excipient physiologiquement acceptable, au moins un dérivé de fluorophénacyl-amine selon l'invention ou l'un de ses sels d'addition non toxiques, l'ingrédient actif étant bien entendu administré à une dose pharmaceutiquement efficace.

Des essais comparatifs ont été entrepris pour mettre en évidence quels sont les effets chez l'animal qui permettent de distinguer les produits selon l'invention de leurs homologues fluorés connus. Parmi ces essais, on a résumé ceux concernant les propriétés antiagressives. Plus précisément, on a évalué la diminution de l'agressivité intergroupes selon la technique suivante : après avoir séjourné pendant 3 semaines de par et d'autre d'une cloison opaque séparant leur cage par le milieu, des groupes de trois souris mâles de 20 g chacune environ reçoivent, par voie intrapéritonéale, les produits à tester en solution dans de l'eau distillée, les animaux témoins ne recevant que de l'eau distillée par voie intrapéritonéale. Une demi-heure plus tard, les deux groupes d'une même cage sont réunis par retrait de la cloison opaque et on note le nombre de combats qui surviennent en 10 min. On a utilisé trois cages pour chaque produit à tester et six cages pour le lot témoin ne recevant pas les produits à tester.

Les résultats du tableau I ci-après donnent la diminution de l'agressivité intergroupes par rapport au lot témoin, tous les produits à tester étant administrés à la dose de 8 mg/kg par voie intrapéritonéale. Ces résultats démontrent :

(i) que les produits selon l'invention (exemple 1 à exemple 5) ont un effet antiagressif nettement supérieur à celui de leurs homologues connus (CP1 à CP4), et

(ii) qu'il n'y a pas de relation structure-activité.

(Voir Tableau I, page 4)

Tableau I

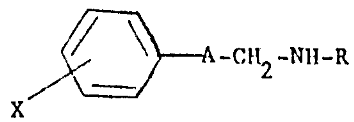

| Produit | n° de code | X | A | R | Dose mg/kg | Diminution de l'agressivité intergroupes |
|---|---|---|---|---|---|---|
| Exemple 1 (a) | CRL 40727 | 4-F | CO | $CH(CH_3)_2$ | 8 | 75% |
| Exemple 2 (a) | CRL 40828 | 2-F | CO | $C(CH_3)_3$ | 8 | 61% |
| Exemple 3 (a) | CRL 40827 | 2-F | CHOH | $C(CH_3)_3$ | 8 | 76% |
| Exemple 4 (b) | CRL 40827A | 2-F | CHOH | $C(CH_3)_3$ | 8 | 78% |
| Exemple 5 (a) | CRL 40854 | 4-F | CHOH | $C(CH_3)_3$ | 8 | 69% |
| CP1 (a) (c) | - | 3-F | CHOH | $CH(CH_3)_2$ | 8 | 5% |
| CP2 (a) (c) | - | 3-F | CHOH | $C(CH_3)_3$ | 8 | 31% |
| CP3 (a) (d) | CRL 40853 | 4-F | CHOH | $CH(CH_3)_2$ | 8 | 40% |
| CP4 (a) (d) | - | 2-F | CHOH | $CH(CH_3)_2$ | 8 | 6% |

Notes (a) : chlorhydrate; (b) : fumarate; (c) : décrit par A.M. LANDS; (d) : décrit par L. VILLA et al.

On a donné ci-après quelques exemples de préparation nullement limitatifs mais à titre d'illustration.

Préparation I

Obtention du chlorhydrate de N-(4-fluorophénacyl)-isopropylamine.

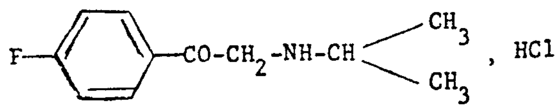

(Exemple 1 ; numéro de code : CRL 40727).

On coule goutte à goutte 25 ml de brome dans une solution, refroidie par un bain de glace, de 69 g (0,5 mole) de parafluoroacétophénone dans 100 ml d'acide acétique. On agite 1 h et on évapore à sec. On reprend le résidu par 100 ml de méthanol et on coule la solution ainsi obtenue dans une solution de 210 ml d'isopropylamine dans 200 ml de méthanol. On porte à reflux 2 h. On évapore à sec. On reprend le résidu à l'eau, on extrait la base libre du produit attendu par l'acétate d'éthyle, sèche le solvant et précipite le chlorhydrate par l'éthanol chlorhydrique. Par recristallisation dans le mélange acétone-méthanol (1 : 1) v/v, on obtient 17,2 g (rendement : 14,8 %) de CRL 40727. F. = 207 °C (avec décomposition).

Analyse % N mesuré : 6,01 %
% N théorique : 6,04 %

Préparation II

Obtention du chlorhydrate de N-(2-fluorophénacyl)-tertiobutylamine.

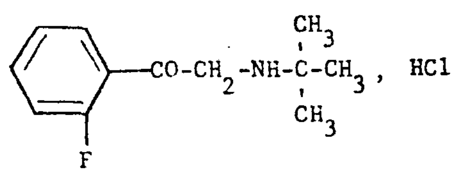

(Exemple 2 ; numéro de code : CRL 40828).

On dissout 50 g (0,362 mole) d'orthofluoroacétophénone dans 75 ml d'acide acétique. On refroidit par un bain de glace et on coule, goutte à goutte, 18,1 ml de brome. On laisse en contact 1 h, évapore à sec et reprend le résidu par 100 ml de méthanol. On coule la solution ainsi obtenue dans une solution de 132 g de tertiobutylamine dans 100 ml de méthanol. On porte à reflux 1 h, évapore à sec, reprend le résidu à l'eau, extrait à l'éther et précipite le chlorhydrate attendu par l'éthanol chlorhydrique. Par recristallisation dans le mélange acétone-éthanol (1 : 1) v/v, on obtient 18 g (rendement = 20 %) de CRL 40828. F. = 240 °C (avec décomposition).

Analyse % N mesuré : 5,74 %
% N théorique : 5,70 %

Préparation III

Obtention du chlorhydrate de 1-(2-fluorophényl)-2-tertiobutylamino-1-éthanol.

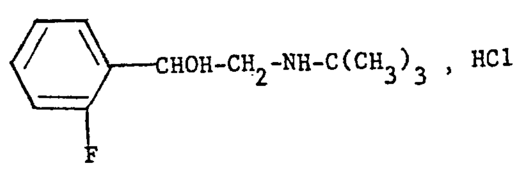

(Exemple 3 ; numéro de code : CRL 40827).

On dissout 0,04 mole de N-(2-fluorophénacyl)-tertiobutylamine (base libre du CRL 40828) dans 120 ml de méthanol. On refroidit à − 5 °C et ajoute 3 g de borohydrure de sodium. On laisse en contact pendant 1 h. On détruit l'excès de $NaBH_4$ restant dans le milieu réactionnel au moyen de 5 ml d'acide acétique, puis on évapore à sec. On reprend le résidu d'évaporation avec de l'eau, ajuste à pH 11 au moyen de NaOH, extrait à l'éther, lave la phase éthérée à l'eau et sèche ladite phase éthérée sur $MgSO_4$. Après filtration, on recueille la base libre, puis on précipite le chlorhydrate attendu au moyen d'éthanol chlorhydrique. Par recristallisation dans le mélange acétone-éthanol (1 : 1) v/v, on obtient 8 g (rendement = 80 %) de CRL 40827. F. = 180,5 °C.

Analyse % N mesuré : 5,60 %
% N théorique : 5,65 %

Préparation IV

Obtention du fumarate de 1-(2-fluorophényl)-2-tertiobutylamino-1-éthanol.

(Exemple 4 ; numéro de code CRL 40827A).

En faisant réagir le 1-(2-fluorophényl)-2-tertiobutyl-amino-1-éthanol (base libre obtenue dans la préparation III) avec l'acide fumarique, on obtient le CRL 40827A. F = 195-200 °C (avec décomposition).

Préparation V

Obtention du chlorhydrate de 1-(4-fluorophényl)-2-tertiobutylamino-1-éthanol.

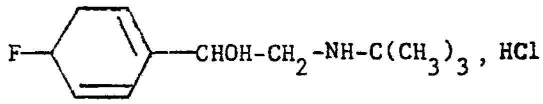

(Exemple 5 ; numéro de code CRL 40854).

On dissout 50 g (0,289 mole) d'α-chloro-p-fluoroacétophénone dans 900 ml de méthanol. On refroidit à − 5 °C et on ajoute 5,80 g de $NaBH_4$. On laisse en contact pendant 1 h, puis ajoute 10 ml d'acide acétique. On ajoute 151 ml de tertiobutylamine et on porte au reflux pendant 12 h. On évapore à sec et reprend le résidu d'évaporation avec de l'eau distillée. On filtre la base libre qui a cristallisé et, par recristallisation dans l'hexane, on obtient 39 g (rendement : 63 % de 1-(4-fluorophényl)-2-tertiobutylamino-1-éthanol. F. = 117 °C.

On dissout cette base dans l'éther diéthylique, précipite le chlorhydrate au moyen d'éthanol chlorhydrique. Par filtration et séchage sous vide sur $P_2O_5$, on obtient 44 g (rendement : 61 %) de CRL 40854. F. = 176 °C.

Le CRL 40854 peut être également préparé selon le procédé de la préparation III en remplaçant la N-(2-fluorophénacyl)-tertiobutylamine par la N-(4-fluorophénacyl)-tertiobutylamine.

On a résumé ci-après les essais qui ont été entrepris avec les produits selon l'invention.

A) Essais relatifs au CRL 40727 (exemple 1)

1. Toxicité

La dose maximale non mortelle, DL-O, est supérieure à 128 mg/kg et inférieure à 256 mg/kg, chez la souris, par voie I.P.

2. Action sur le SNC

Le CRL 40727 présente un certain nombre d'effets de type sédatif, à savoir :

— sédation et hyporéactivité chez la souris,
— hypomotilité et diminution de l'agressivité chez la souris,
— hypothermie et potentialisation des effets hypothermisants de l'apomorphine, l'oxotrémorine et la réserpine,
— antagonisme modéré des stéréotypies amphétaminiques.

3. Action sur le système cardiovasculaire

a) par voie intraveineuse

Deux chiens reçoivent le CRL 40727 par voie intraveineuse, en perfusion de 6 min, aux doses successives de 0,1 mg/kg, 1 mg/kg, 2,5 mg/kg, 5 mg/kg, 10 mg/kg et 20 mg/kg. On mesure leur pression artérielle, leur fréquence cardiaque, leur débit fémoral, leur température rectale.

On observe ce qui suit.

Le CRL 40727 augmente le débit fémoral à partir de la dose de 1 mg/kg ; l'effet augmente avec la dose, jusqu'à 10 mg/kg, dose pour laquelle on atteint + 140 %.

A partir de 5 mg/kg, la pression artérielle différentielle augmente ; les pressions artérielles diastolique et moyenne diminuent à partir de 10 et, respectivement, 20 mg/kg.

La fréquence cardiaque n'est pas nettement modifiée.

La peau devient rose à partir de 2,5 à 10 mg/kg.

Le liquide biliaire reste jaune ; la température rectale n'est pas modifiée.

La tachycardie à l'isoprénaline est diminuée, la fréquence cardiaque passe en moyenne à 182 battements/min après 10 mg/kg, alors qu'elle atteignait 215 battements/min en témoin : l'hypotension n'est pas modifiée.

Une expérience complémentaire a été entreprise : un chien reçoit une dose supplémentaire de 40

mg/kg I.V. de CRL 40727, et on observe une hypotension plus importante qu'à la dose précédente, le liquide biliaire restant jaune ; l'autre chien reçoit un produit de référence, le chlorhydrate de (2,4,6-triméthoxyphényl)-(3-pyrrolidinopropyl)-cétone — qui est décrit dans le brevet britannique n° 1 325 192, a pour numéro de code LL 1656 et est commercialisé sous la désignation de FONZYLANE — à la dose de 6 mg/kg I.V., et on observe que le débit fémoral n'augmente pas plus qu'avec la dose de 10 mg/kg de CRL 40727.

b) par voie intraduodénale

Trois chiens reçoivent le CRL 40727 par voie intraduodénale aux doses successives de 1 mg/kg, 2,5 mg/kg, 5 mg/kg et 10 mg/kg. On mesure les mêmes paramètres que ci-dessus. On observe ce qui suit.

Le CRL 40727 augmente nettement le débit fémoral à partir de la dose de 2,5 à 5 mg/kg ; l'effet n'augmente que peu avec la dose. A partir de 10 mg/kg, une action hypotensive se manifeste. La peau rosit très légèrement à partir de 2,5 mg/kg.

Le liquide biliaire reste jaune. La température rectale n'est pas modifiée.

La tachycardie à l'isoprénaline est diminuée. La fréquence cardiaque passe, en moyenne à 165 battements/min après 10 mg/kg de CRL 40727, alors qu'elle atteignait 220 battements/min en témoin. L'hypotension n'est pas modifiée.

Des essais complémentaires ont été entrepris : un chien reçoit une dose supplémentaire de 20 mg/kg-I.D. On observe une action hypotensive plus importante, sans effet vasodilatateur supplémentaire. Le même résultat est obtenu sur un autre chien auquel sont injectés 5 mg/kg I.V. de CRL 40727 en fin d'essai. De plus, le LL 1656 injecté ensuite par voie intraveineuse, à la dose de 6 mg/kg, n'entraîne pas de vasodilatation supplémentaire.

En conclusion, les résultats obtenus par voie intraveineuse et par voie intraduodénale sont difficilement comparables, l'hypotension ne survenant chez les chiens traités par voie intraveineuse qu'à partir de 20 mg/kg, alors qu'elle apparaît avec la même intensité chez les chiens traités par voie intraduodénale à 10 mg/kg.

L'action vasodilatatrice du CRL 40727 est peut-être due à une action $\beta_2^+$ ; aucune action $\beta_1^+$ n'est observée (pas de tachycardie), aucune bradycardie, par contre, l'action tachycardisante de l'isoprotérénol est diminuée. De plus, on note que le liquide biliaire reste jaune même après la dose cumulée de 38,5 mg/kg I.V.

B) Essais relatifs au CRL 40827 (exemple 3)

Le CRL 40827, en solution dans de l'eau distillée, a été administré par voie intrapéritonéale sous un volume de 20 ml/kg chez la souris mâle et un volume de 5 ml/kg chez le rat mâle.

1. Toxicité

La dose maximale non mortelle, DL-O, est supérieure à 64 mg/kg et inférieure à 128 mg/kg chez la souris mâle.

2. Action sur le SNC

Interaction avec l'apomorphine

a) souris

Des lots de 6 souris reçoivent le CRL 40827 30 min avant l'injection sous-cutanée d'apomorphine à la dose de 1 ou 16 mg/kg. On observe qu'aux doses de 0,5 mg/kg et 2 mg/kg et surtout 8 et 32 mg/kg le CRL 40727 s'oppose nettement à l'action hypothermisante de la forte dose d'apomorphine, mais ne modifie pas le comportement de verticalisation et les stéréotypies.

b) rat

Le CRL 40 827 est administré à des lots de 6 rats 30 min avant l'injection sous-cutanée de 0,5 mg/kg d'apomorphine. On observe que le CRL 40827 laisse inchangées les stéréotypies induites par l'apomorphine chez le rat.

Interaction avec l'amphétamine

L'amphétamine (2 mg/kg) est injectée par voie intrapéritonéale à des lots de 6 rats, 30 min après l'administration de CRL 40827. On note que, à l'exception de la diminution isolée de l'index de stéréotypies, observée à la dose de 4 mg/kg, le CRL 40827 ne modifie pas les stéréotypies amphétaminiques.

Interaction avec la réserpine

Quatre heures après l'injection intrapéritonéale de 2,5 mg/kg de réserpine, des lots de 6 souris reçoivent le CRL 40827. On constate que, dès la dose de 0,5 mg/kg, le CRL 40827 combat nettement l'hypothermie réserpinique sans modifier le ptôsis.

Interaction avec l'oxotrémorine

Le CRL 40827 est administré à des lots de 6 souris 30 min avant l'injection intrapéritonéale de 0,5 mg/kg d'oxotrémorine. On observe que, dès la dose de 0,5 mg/kg, le CRL 40827 antagonise l'action hypothermisante de l'oxotrémorine ; cet effet est très net à 32 mg/kg. De plus, le CRL 40827 ne modifie pas l'intensité des tremblements provoqués par l'oxotrémorine. Enfin, le CRL 40827 laisse inchangés les signes de stimulation cholinergique périphérique qui apparaissent après administration d'oxotrémorine.

Action sur le test des quatre plaques, la traction et l'électrochoc

Le test est pratiqué sur des lots de 10 souris, 30 min après l'administration de CRL 40827. Le CRL 40827 n'entraîne pas d'augmentation du nombre de passages punis ; il ne provoque pas d'incapacité motrice majeure et, à forte dose, s'oppose aux effets convulsivants de l'électrochoc.

Action sur la motilité spontanée

Une demi-heure après avoir reçu le CRL 40827, les souris (6 par dose, 12 témoins) sont placées en actimètre où leur motilité est enregistrée pendant 30 min. On observe que le CRL 40827 ne modifie pratiquement pas l'activité motrice spontanée de la souris.

Action vis-à-vis de quelques comportements perturbés par divers agents

a) Motilité réduite par habituation à l'enceinte

Après 18 h de séjour dans des actimètres, les souris (6 par dose, 12 témoins) reçoivent le CRL 40827. Elles sont aussitôt replacées dans leurs enceintes respectives et, 30 min plus tard, on enregistre leur motilité pendant 30 min. A la forte dose (34 mg/kg), le CRL 40827 semble provoquer une reprise modérée de l'activité motrice.

b) Motilité réduite par agression hypoxique

Une demi-heure après avoir reçu le CRL 40827, les souris (10 par dose, 20 témoins) sont soumises à une anoxie hypobare aiguë (dépression de $8 \times 10^4$ Pa en 90 s, détente de 45 s), puis elles sont placées en actimètre où leur motilité est enregistrée pendant 10 min. On note que le CRL 40827 n'entraîne pas d'amélioration de la récupération motrice chez les souris dont la motilité a été déprimée à la suite d'un séjour bref dans une enceinte à pression réduite.

c) Anoxie asphyxique

Des lots de 10 souris reçoivent le CRL 40827 30 min avant l'administration intrapéritonéale d : 34 mg/kg de triiodoéthylate de gallamine. A la plus forte dose (34 mg/kg), le CRL 40827 empêche l'apparition des convulsions et de la mort chez 40 % des animaux.

Conclusion relative à l'action sur le SNC

L'antagonisme des hypothermies induites par l'apomorphine, la réserpine ou l'oxotrémorine permet de prévoir une activité du type antidépresseur pour le CRL 40827. Ces antagonismes étant observés en l'absence d'effet anticholinergique, le CRL 40827 se différencie donc des imipraminiques.

D'autre part, l'absence d'antagonisme du ptôsis réserpinique et de stimulation motrice avec stéréotypies permet de distinguer le CRL 40827 des IMAO et, respectivement, des amphétaminiques. En bref, il y a de fortes présomptions que le CRL 40827 se comporte comme les stimulants β-adrénergiques.

Par ailleurs, le CRL 40827 exerce uniquement, à forte dose, des effets anticonvulsivants. Enfin, il diminue l'agressivité intergroupes chez la souris.

3. Action sur le système cardiovasculaire et respiratoire

On observe que le CRL 40827 agit en tant qu'agent hypotenseur et tachycardisant chez le chien anesthésié et chez le rat éveillé génétiquement hypertendu, qu'il diminue les résistances vasculaires des territoires explorés (vertébral, fémoral et rénal) et la résistance périphérique totale, qu'il diminue le travail du ventricule gauche et raccourcit la diastole, qu'il stimule la respiration, qu'il diminue les effets hypertenseurs de la noradrénaline à partir de la dose de 1 mg/kg chez le chien (l'effet maximal étant atteint à la dose de 10 mg/kg environ).

Les débits sanguins locaux n'augmentent pas mais les résistances diminuent, ce phénomène impliquerait que le CRL 40827 induit une vasodilatation périphérique artérielle en même temps qu'une augmentation du retour veineux, puisque le débit cardiaque reste égal.

4. Action sur la sécrétion biliaire

Chez le chien anesthésié au Nembutal, le débit biliaire normal recueilli en 30 min est de 1,5 ml ; le débit biliaire augmente après injection intraduodénale de CRL 40827 et passe à 1,75 ml pour 2,5 mg/kg de

CRL 40827, à 2,25 ml pour 5 mg/kg et à 2,5 ml pour 10 mg/kg. Chez le rat anesthésié au Nembutal, le débit biliaire augmente 1 à 3 h après injection I.V. de CRL 40827 aux doses de 5 mg/kg et 25 mg/kg.

5. Effet anesthésique local

L'effet anesthésique a été étudié chez le cobaye après injection du CRL 40827 par voie intradermique sous un volume de 0,2 ml aux concentrations de 0,1 0,5 et 1 % (3 cobayes par dose). Chaque animal reçoit le sérum physiologique, la procaïne et le CRL 40827 dans des zones délimitées.

Le test qui consiste en une série de six piqûres de la zone injectée est réalisé 5, 10, 15, 20, 25 et 30 min après l'injection. On constate que le CRL 40827 présente un effet anesthésique local quand il est administré aux concentrations de 0,5 et 1 %.

C) Essais relatifs au CRL 40854 (exemple 5)

Le CRL 40854, en solution dans de l'eau distillée, a été administré par voie intrapéritonéale sous un volume de 20 ml/kg chez la souris mâle et de 5 ml/kg chez le rat mâle.

1. Toxicité

La dose maximale non mortelle DL-O est supérieure à 128 mg/kg et inférieure à 256 mg/kg chez la souris.

2. Action sur le SNC

En procédant selon les modalités opératoires données ci-dessus pour le CRL 40827, on observe ce qui suit.

Interaction avec l'apomorphine

Aux doses de 16 et 64 mg/kg chez la souris, le CRL 40854 s'oppose modérément à l'action hypothermisante de l'apomorphine sans modifier le comportement de verticalisation et les stéréotypies.

Chez le rat, le CRL 40854 ne modifie pas les stéréotypies induites par l'apomorphine.

Interaction avec l'amphétamine

Aux doses de 8 et 32 mg/kg, le CRL 40854 potentialise la durée des stéréotypies amphétaminiques.

Interaction avec la réserpine

Aux doses de 4,16 et 64 mg/kg, le CRL 40854 antagonise modérément l'hypothermie réserpinique sans modifier le ptôsis.

Interaction avec l'oxotrémorine

Aux doses de 10 et 64 mg/kg, le CRL 40854 aggrave l'effet hypothermisant de l'oxotrémorine. Il ne modifie pas les tremblements et les signes de stimulation cholinergique périphérique.

Action sur le test des quatre plaques, la traction et l'électrochoc

Comme le CRL 40827, le CRL 40854 n'entraîne pas d'augmentation du nombre de passages punis et ne provoque pas de déficit moteur majeur. En revanche, il ne modifie pas les effets convulsivants de l'électrochoc.

Action sur la motilité spontanée.

A forte dose (64 mg/kg), le CRL 40854 diminue modérément la motilité spontanée de la souris.

Action vis-à-vis de quelques comportements perturbés par divers agents

a) Motilité réduite par habituation à l'enceinte

Le CRL 40854 ne provoque pas de reprise nette de l'activité motrice chez la souris habituée à son enceinte.

b) Motilité réduite par agression hypoxique

Comme le CRL 40827, le CRL 40854 n'entraîne pas d'amélioration de la récupération motrice chez la souris.

c) Anoxie asphysique

Le CRL 40854 ne modifie pas l'apparition des convulsions et la mort consécutives à une anoxie provoquée par curarisation.

En conclusion, le CRL 40854, qui diminue l'agressivité intergroupes chez la souris, présente un ensemble de résultats surprenants par rapport aux agents sédatifs et antidépresseurs classiques.

En clinique, on a obtenu de bons résultats avec le CRL 40727, le CRL 40827 et le CRL 40827A dans le domaine psychotrope en tant qu'agents sédatifs et antidépresseurs. En particulier, les CRL 40727 et CRL 40827A ont donné chacun d'excellents résultats en tant qu'agents antidépresseurs chez l'homme après avoir été administrés sous forme de comprimés ou de gélules renfermant chacun 5 mg de principe actif, à raison de 3 comprimés ou gélules par jour.

**Revendications** (pour les Etats contractants : BE, CH, DE, GB, IT, LI, LU, NL, SE)

1. Nouveau composé appartenant à la famille des dérivés de fluorophénacyl-amine répondant à la formule générale

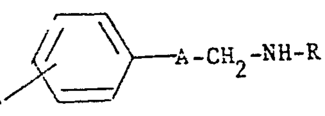

(I)

où A représente CO ou CHOH, et R est $CH(CH_3)_2$ ou $C(CH_3)_3$, ledit composé étant caractérisé en ce qu'il est choisi parmi l'ensemble constitué par les N-(4-fluorophénacyl)-isopropylamine, N-(2-fluorophénacyl)-tertiobutylamine, 1-(2-flurophényl)-2-tertiobutylamino-1-éthanol, 1-(4-fluorophényl)-2-tertiobutylamino-1-éthanol, et leurs sels d'addition.

2. N-(4-Fluorophénacyl)-isopropylamine et ses sels d'addition.

3. N-(2-(Fluorophénacyl)-tertiobutylamine et ses sels d'addition.

4. 1-(2-Fluorophényl)-2-tertiobutylamino-1-éthanol et ses sels d'addition.

5. 1-(4-Fluorophényl)-2-tertiobutylamino-1-éthanol et ses sels d'addition.

6. Composition thérapeutique caractérisée en ce qu'elle renferme, en association avec un excipient physiologiquement acceptable, au moins un dérivé de fluorophénacyl-amine selon la revendication 1 ou l'un de ses sels d'addition non toxiques.

**Revendication** (pour l'Etat contractant AT)

Procédé de préparation d'un dérivé appartenant à la famille des fluorophénacyl-amines de formule générale

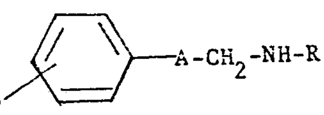

(I)

où A est CO ou CHOH et R est $CH(CH_3)_2$ ou $C(CH_3)_3$, et choisi parmi l'ensemble constitué par les N-(4-fluorophénacyl)-isopropylamine, N-(2-fluorophénacyl)-tertiobutylamine, 1-(2-fluorophényl)-2-tertiobutyl-amino-1-éthanol, 1-(4-fluorophényl)-2-tertiobutylamino-1-éthanol, et leurs sels d'addition, ledit procédé étant caractérisé en ce que

1. on fait réagir un halogénure de fluorophénacyle de formule

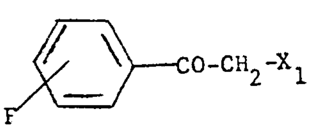

(II)

où $X_1$ est Cl ou Br, avec une amine de formule

$$H_2NR \quad (III)$$

où R est défini comme ci-dessus dans un alcool, pendant au moins 1 heure à la température de reflux du milieu réactionnel, pour obtenir un dérivé de formule I où A est CO ; et,

2. le cas échéant, on réduit le dérivé carbonyle où A est CO ainsi obtenu, au moyen de $NaBH_4$ pour obtenir un dérivé de formule I où A est CHOH.

**Claims** (for the Contracting States : BE, CH, DE, GB, IT, LI, LU, NL, SE)

1. A new compound belonging to the family of fluorophenacyl-amine derivatives of the formula

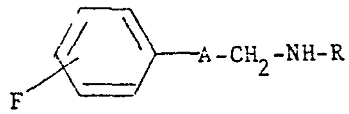

(I)

[wherein A is CO or CHOH and R is $CH(CH_3)_2$ or $C(CH_3)_3$] said compound being characterized in that it is selected from the group consisting of N-(4-fluorophenacyl)-isopropylamine, N-(2-fluorophenacyl)-tertiobutylamine, 1-(2-fluorophényl)-2-tertiobutylamino-1-ethanol, 1-(4-fluorophenyl)-2-tertiobutylamino-1-ethanol and their addition salts.

2. N-(4-Fluorophenacyl)-isopropylamine and its addition salts.

3. N-(2-Fluorophenacyl)-tertiobutylamine and its addition salts.

4. 1-(2-Fluorophenyl)-2-tertiobutylamino-1-ethanol and its addition salts.

5. 1-(4-Fluorophenyl)-2-tertiobutylamino-1-ethanol and its addition salts.

6. A therapeutical composition characterized in that it contains, in association with a physiologically acceptable excipient, at least fluorophenacyl-amine derivate according to claim 1, or one of its non-toxic addition salts.

**Claim** (for the Contracting State AT)

Process for the preparation of a derivative belonging to the family of fluorophenacylamine derivatives of the formula

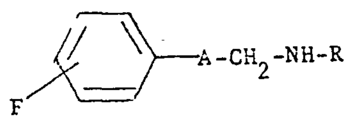

(I)

wherein A is CO or CHOH and R is $CH(CH_3)_2$ or $C(CH_3)_3$ selected from the group consisting of N-(4-fluorophenacyl)-isopropylamine, N-(2-fluorophenacyl)-tertiobutylamine, 1-(2-fluorophenyl)-2-tertiobutylamino-1-ethanol, 1-(4-fluorophenyl)-2-tertiobutylamino-1-ethanol and their addition salts, said process being characterized in that

1. a fluorophenacyl halide of formula

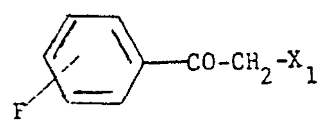

(II)

wherein $X_1$ is Cl or Br, is reacted with an amine of formula

$H_2NR$ (III)

wherein R is defined as above in an alcohol, for at least one hour at the reflux temperature of the reaction medium, to obtain a derivative of formula I wherein A is CO, and

2. the carbonyl derivative wherein A is CO thus obtained is reduced by means of $NaBH_4$ to obtain a derivative of formula I wherein A is CHOH.

**Ansprüche** (für die Vertragsstaaten : BE, CH, DE, GB, IT, LI, LU, NL, SE)

1. Neue, der Familie der Fluorphenacylamin-Derivate angehörende Verbindung der allgemeinen Formel

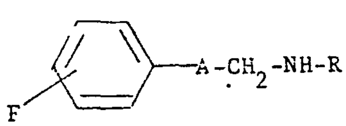

(I)

worin A CO oder CHOH bedeutet und R $—CH(CH_3)_2$ oder $—C(CH_3)_3$ darstellt, dadurch gekennzeichnet, daß die Verbindung ausgewählt ist aus der Gruppe bestehend aus N-(4-Fluorphenacyl)-isopropylamin, N-(2-Fluorphenacyl)-tert.butylamin, 1-(2-Fluorphenyl)-2-tert.butylamino-1-äthanol, 1-(4-Fluorphenyl)-2-tert.butylamino-1-äthanol und ihre Additionssalze.

2. N-(4-Fluorphenacyl)-isopropylamin und seine Additionssalze.

3. N-(2-Fluorphenacyl)-tert.butylamin und seine Additionssalze.

4. 1-(2-Fluorphenyl)-2-tert.butylamino-1-äthanol und seine Additionssalze.

5. 1-(4-Fluorphenyl)-2-tert.butylamino-1-äthanol und seine Additionssalze.

6. Therapeutische Zusammensetzung, dadurch gekennzeichnet, daß sie in Verbindung mit einem physiologisch akzeptablen Exzipienten wenigstens ein Fluorphenacylamin-Derivat nach Anspruch 1 oder eines von dessen nicht toxischen Additionssalzen enthält.

**Anspruch** (für den Vertragsstaat AT)

Verfahren zur Herstellung eines der Familie der Fluorphenacylamine angehörenden Derivates der allgemeinen Formel

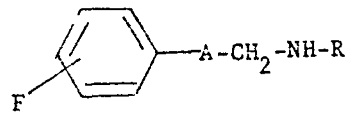

(I)

worin A CO oder CHOH bedeutet und R $-CH(CH_3)_2$ oder $-C(CH_3)_3$ darstellt, ausgewählt aus der Gruppe bestehend aus N-(4-Fluorphenacyl)-isopropylamin, N-(2-Fluorphenacyl)-tert.butylamin, 1-(2-Fluorphenyl)-2-tert.butylamino-1-äthanol, 1-(4-Fluorphenyl)-2-tert.butylamino-1-äthanol und ihren Additionssalzen, dadurch gekennzeichnet, daß man

1. zur Herstellung eines Derivates der Formel (I), worin A CO bedeutet, ein Fluorphenacylhalogenid der Formel

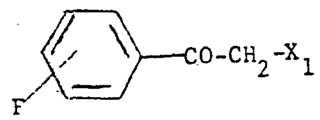

(II)

worin $X_1$ für Chlor oder Brom steht, mit einem Amin der Formel

$$H_2NR \qquad (III)$$

worin R wie oben definiert ist, in einem Alkohol während wenigstens einer Stunde bei der Rückflußtemperatur der Reaktionsmischung reagieren läßt, und

2. gegebenenfalls das so erhaltene Carbonylderivat, worin A CO bedeutet, mittels $NaBH_4$ reduziert, um ein Derivat der Formel (I), worin A für CHOH steht, zu erhalten.